# EUROPEAN PATENT APPLICATION

(11) **EP 1 495 734 A1**
(43) Date of publication of application: **12.01.2005**
(21) Application number: 04076979.6
(22) Date of filing: 08.07.2004
(51) Int. Cl.: A61B 18/02

(54) **Method for providing a cryogenic applicator, and assembly of at least one applicator and a cooling device**

(30) Priority: 09.07.2003 NL 1023870
(71) Applicant: Recticel Nederland B.V., 4041 CL Kesteren (NL)
(72) Inventor: Lievestro, Robert, 6662 CC Elst (NL); Jansen, Lambertus, 4061 BZ Ophemert (NL)
(74) Representative: Winckels, Johannes Hubertus F.

(57) **Abstract**

A method for providing a cryogenic applicator, which applicator (1) comprises at least one plastic foam part (2), wherein the foam part (2) is at least partly compressed to a particular treatment shape and simultaneously cooled, such that the foam part (2) is stiffened in the treatment shape for a particular treatment period. In addition, the invention provides an assembly of at least one applicator and a cooling device, wherein the applicator (1) comprises at least one plastic foam part (2), the device (11) being provided with a cooling chamber to cool the foam part (2) of the applicator (1), at least when the foam part (2) has been placed in a cooling position in the cooling chamber, the cooling chamber comprising a shaping cavity (12) to compress the foam part (2) at the cooling position at least partly to a particular treatment shape.

## Description

The invention relates to a method for providing a cryogenic applicator, which applicator comprises at least one plastic foam part.

Such a method is known from Dutch patent no. 1010774. In the known method, a foam part of an administration element is cooled by introducing the foam part into a cooling chamber of a cooling device, which chamber is subsequently filled with an amount of liquid cooling agent, so that the foam can be soaked with cooling agent. The thus cooled foam part can be used, for instance, for a cryogenic surface treatment. The method known from NL 1010774 is intended in particular to perform a cosmetic treatment of the skin, for instance to remove a wart, without this requiring special medical know-how of a medical specialist.

The foam part of the applicator known from NL 1010774 is cylinder-shaped and has a relatively open cell structure. An advantage is that such a foam part is relatively easy to manufacture. Moreover, such a foam part can absorb relatively much cooling agent, so that with the foam part a thorough, relatively long cryogenic treatment penetrating relatively deeply into a surface can be performed. However, a disadvantage of the foam part of that known applicator is that the terminal end of the foam part provides a relatively large, flat treatment surface with which no accurate treatment can be performed.

European patent application EP 0 608 954 A1 discloses a method in which a foam body of an administration element is cooled in order to subsequently treat a surface cryogenically with it. In this known method, a treatment surface of the foam body is first pressed onto a surface to be treated cryogenically. Next, cooling agent is supplied to the foam body, such that the desired cryogenic treatment takes place. According to EP 0 608 954, the shape of the foam body can be adapted to the use to be carried out with it, for instance by the use of foam bodies having tapering or rounded ends. A drawback of such foam bodies is that they are relatively difficult to manufacture. From practice, it is known to form these foam bodies thermally, at a relatively high temperature, which takes relatively much energy and adversely affects the properties of the foam. For instance, thermoforming leads to a relatively closed cell structure. Such a cell structure has a relatively poor ability to absorb and release an amount of cooling agent desired for cryogenic treatment. Such a thermoformed administration element is therefore not capable of performing a desired efficient, thorough surface treatment. A further disadvantage of the method described in EP 0 608 954 is that during the cryogenic treatment unduly much cooling agent must be used to cool the foam part placed on the surface to be treated.

The object of the present invention is to eliminate the above-mentioned problems. In particular, the object of the invention is to provide a method according to the opening paragraph hereof, by which an applicator of a shape suitable for cryogenic treatment is provided in a simple manner.

To that end, the method is characterized according to the invention by the features of claim 1.

The foam part is at least partly compressed to a particular treatment shape and simultaneously cooled, such that the foam part is stiffened in that treatment shape for a particular treatment period.

Plastic foam, which is normally relatively soft and compressible at room temperature, can stiffen below a particular temperature. The inventive idea underlying the invention is that the cryogenic stiffening of the foam is easily usable to give the foam the desired treatment shape. Moreover, the foam part can yet be made of relatively inexpensive design, with an open cell structure that is advantageous for the treatment. Upon stiffening, the foam can further preserve that open cell structure, which is particularly advantageous for the effectiveness of the cryogenic treatment to be performed with it.

The formation of the foam part is accompanied by the cooling of the foam. After this cryogenic formation, the foam retains the treatment shape for a particular treatment period, for the purpose of a treatment to be performed with it. It has been found that the plastic foam can easily preserve this treatment shape for a relatively long treatment period, which is, for instance, at least about 10 seconds. The length of the period of form retention naturally depends *inter alia* on the kind of foam, dimensions of the foam body, the temperature obtained by the foam during cooling, as well as on the ambient temperature.

Furthermore, the invention provides a method for the cosmetic treatment of a skin part, which method is characterized by the features of claim 7. A foam part of an applicator is first stiffened in a treatment shape through cooling by means of a method according to any one of claims 1-6. The stiffened, cooled foam part is subsequently pressed with a respective treatment surface onto the skin part. In this way, a relatively accurate and thorough cosmetic treatment of the skin part can be obtained.

The invention further relates to an assembly of at least one applicator and a cooling device, the applicator comprising at least one plastic foam part, and the device being provided with a cooling chamber to cool the foam part of the applicator, at least when the foam part has been placed in a cooling position in the cooling chamber.

Such an assembly is likewise known from the Dutch patent no. 1010774 referred to. Disadvantages of this assembly have been mentioned hereinabove. An object of the invention is to improve this assembly, in particular to provide the applicator with a desired treatment shape in a simple manner. To that end, according to the invention, the device is characterized in that the cooling chamber comprises a shaping cavity to compress the foam part at the cooling position at least partly to a particular treatment shape. In a simple manner, the foam part can be simply pressed into the desired shape and be cooled to be frozen in that shape, which provides the above-mentioned advantages.

Presently, the invention will be further elucidated with reference to an exemplary embodiment and the drawing. In the drawing:
Fig. 1 shows a schematically represented cross-sectional view of an exemplary embodiment of the invention, with the foam part of the applicator situated outside the cooling chamber;
Fig. 2 shows a similar view to Fig. 1, with the foam part of the applicator placed in a cooling position in the cooling chamber; and
Fig. 3 shows three different designs of an applicator according to the invention.

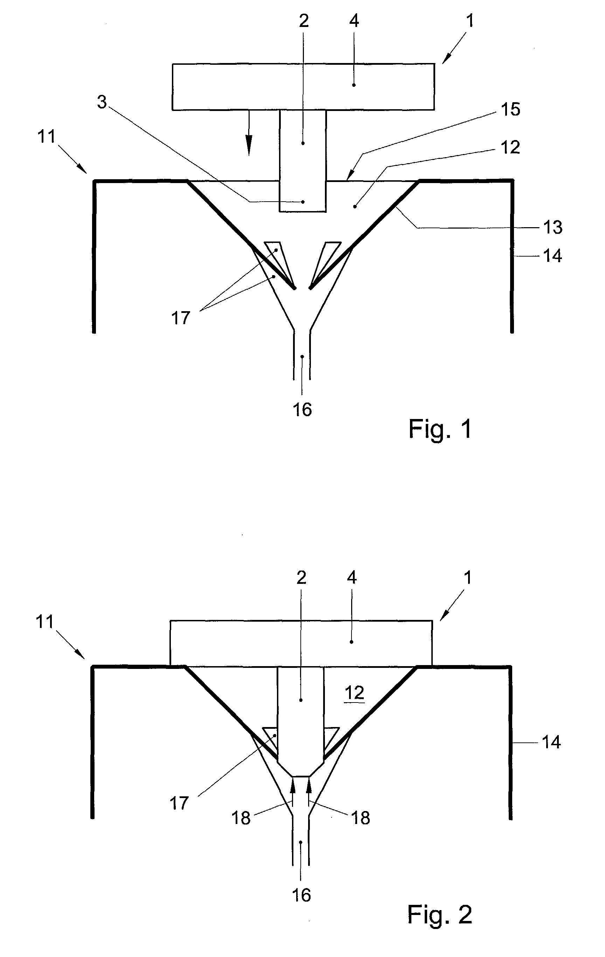

Fig. 1 shows an assembly of an applicator 1 and a cooling device 11. The applicator 1 is provided with a plastic foam part 2 which extends at right angles to a holder 4. The foam 2 is compressible, at least in an uncooled condition at room temperature. The foam part 2 is further substantially cylinder-shaped, at least in an uncompressed condition. The holder 4 of the applicator 1 serves as a grip by which the applicator can be held. Further, the holder 4 forms a closing member to substantially fluid-tightly close off an access to the cooling chamber 13 of the cooling device 11 shown, see Fig. 2.

A free end 3 of the foam part 2 forms a treatment end, with which, after cooling in the cooling chamber 13, a surface treatment can be performed. According to the invention, it is particularly advantageous when at least the treatment part 3 of the foam part has an open cell structure for good absorption and release of cooling agent. The foam has for instance a Cell Dimension (CD) of 400-2,000 microns, which corresponds to about 60-100 Pores Per Inch (PPI). The density of the foam is, for instance, about 18-75 kg/m³, more particularly about 58-60 kg/m³.

The cooling device 11 is provided with a housing 14, of which an outer wall bounds a cooling chamber 13. The cooling chamber 13 comprises in particular a shaping cavity 12. The shaping cavity 12 is arranged to press the treatment end 3 of the foam part 2 into a pointed shape when the foam part 2 has been placed in a cooling position in the cooling chamber 13. To that end, the shaping cavity 12 is of conical design.

Via a passage 15 of the housing 14, the cooling chamber 13 is accessible from an environment to press the foam part 2 of an applicator 1 into the shaping cavity 12. The cooling device 11 furthermore comprises releasing means with supply means 16, 17 to supply a cooling fluid to the cooling chamber 13. These means comprise a supply channel 16 which terminates in the apex of the conical shaping cavity 12. The supply channel 16 moreover branches off into a number of side channels that terminate in the shaping cavity 12 via longitudinal slits 17. With these supply means 16, 17, the shaping cavity 12 can be operatively filled with a cooling agent in a relatively homogeneous manner. The fluid supply means 16, 17 are connected to a fluid reservoir, not shown. Further, the releasing means are provided with operating means, not shown, for instance operable valve means or the like, to allow fluid to flow in a controlled manner from the reservoir via supply means 16, 17 to the shaping cavity 12. The cooling device can comprise, for instance, an aerosol can or the like.

For the purpose of use, the fluid reservoir is filled with a suitable cooling fluid to cool the foam part 2 to such a temperature that the foam stiffens. This temperature can be, for instance, about -40°C or lower. The cooling fluid is, in particular, liquid. Various substances are suitable as coolant. An environmentally advantageous cooling fluid comprises, for instance, at least dimethyl ether and an alkane, in particular propane.

Figs. 1 and 2 show the use of the assembly for the purpose of providing a cryogenic applicator 1. To that end, the applicator 1 is first positioned, by the holder 4 thereof, onto the cooling device 11, such that the foam part 2 is placed in a cooling position in the cooling chamber 13. It follows from Fig. 2 that the dimensions of the applicator 1 are such that the applicator 1 closes off the access 15 to the cooling chamber 13 at the cooling position by means of the holder 4. As further shown by Fig. 2, the treatment end 3 of the foam part 2 in the cooling position is compressed in the shaping cavity 12 to a pointed shape.

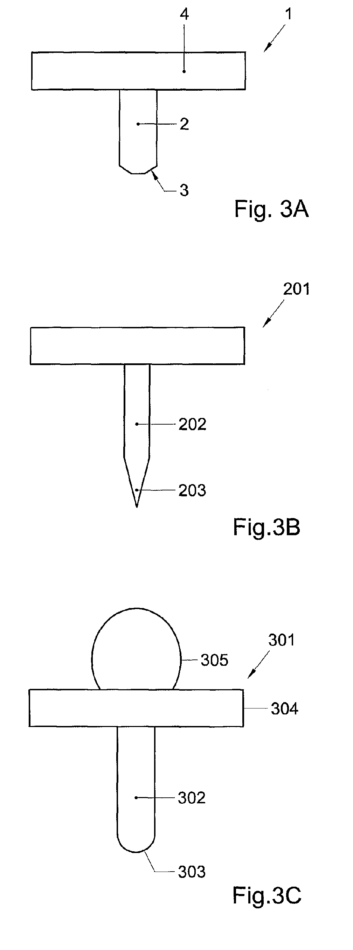

In the thus closed cooling chamber 13, the partly compressed foam part 2 is subsequently cooled by supplying the cooling fluid via the supply means 16, 17 to the cooling chamber 13, which is represented with arrows 18. The holder 4 of the applicator 1 then prevents cooling fluid from undesirably flowing out of the cooling chamber 13. The cooling of the foam part 2 is such that the foam part 2 is stiffened in the pointed shape for a particular treatment period. Next, the applicator 1 with the stiffened foam part 2 can be removed from the cooling chamber 13. The resulting stiffened applicator 1 is represented in Fig. 3A. Next, with the surface of the free treatment end 3, a surface treatment can be performed. Owing to the pointed shape of the treatment end 3, the treatment can be performed accurately and in a focused manner.

Figs. 3B, 3C shows alternative outcomes of an applicator after freezing by a cooling device provided with a suitable shaping cavity. In Fig. 3B, the foam part 202 of the applicator 201 is provided with a relatively sharp treatment tip 203, in order that a very precise treatment can be performed.

Fig. 3C shows a variant in which a side of the holder 304 remote from the foam part 2 is provided with a grip 305 for properly holding the applicator 301. In this case, the end 303 of the foam part 302 has been frozen in a rounded shape.

It will be evident to the skilled person that the invention is not limited to the exemplary embodiments described. Various modifications are possible within the framework of the invention as set forth in the following claims.

For instance, the foam part of the applicator can contain various kinds of foam, for instance one or more different types of foam. The foam part can, for instance, be cooled wholly or partly to retain the treatment shape.

The applicator 1 can have various shapes and dimensions. Thus, different applicators 1 can be used in combination with a single cooling device, while the respective foam parts have slightly different lengths. As a consequence, at the cooling position, one foam part will reach further into the shaping cavity 12 than the other. Accordingly, one foam part can then be compressed more than the other, which ― after cooling ― results in stiffened foam parts having different treatment shapes and/or cell structures.

The cooling fluid can comprise various substances, for instance alkanes, ethers, inert gases, nitrogen or the like.

The holder 4 of the applicator 1 can be manufactured from various materials and be designed in different forms. The holder 4 and the foam part 2 can for instance be detachably coupled to each other, so that different foam parts 2 are usable with a single holder. Further, the foam part 2 and the holder 4 may be integrally connected.

The cooling device 11 and the applicator 1 can for instance be supplied jointly or separately.

## Claims

1. A method for providing a cryogenic applicator, which applicator (1) comprises at least one plastic foam part (2), **characterized in that** said foam part (2) is at least partly compressed to a particular treatment shape and simultaneously cooled, such that the foam part (2) is stiffened in said treatment shape for a particular treatment period.

2. A method according to claim 1, wherein said foam part (2) is compressed to said treatment shape in a shaping cavity (12), wherein a cooling fluid is introduced into said shaping cavity (12) for the purpose of cooling and stiffening the foam part (2).

3. A method according to claim 2, wherein an access to said shaping cavity (12) is closed off substantially fluid-tightly during compression of said foam part (2).

4. A method according to any one of the preceding claims, wherein a free treatment end (3) of said foam part (2) is compressed and stiffened.

5. A method according to any one of the preceding claims, wherein the foam part (2) is at least partly compressed to a pointed shape and stiffened.

6. A method according to any one of the preceding claims, wherein said foam has an open cell structure.

7. A method for the cosmetic treatment of a skin part, wherein a foam part (2) of an applicator (1) is first stiffened in a treatment shape through cooling by means of a method according to any one of the preceding claims, wherein the stiffened, cooled foam part (2) is subsequently pressed with a respective treatment surface thereof onto the skin part.

8. An assembly of at least one applicator and a cooling device, wherein said applicator (1) comprises at least one plastic foam part (2), the device (11) being provided with a cooling chamber to cool the foam part (2) of the applicator (1), at least when the foam part (2) has been placed in a cooling position in the cooling chamber, **characterized in that** the cooling chamber comprises a shaping cavity (12) to compress said foam part (2) at said cooling position at least partly to a particular treatment shape.

9. An assembly according to claim 8, wherein the applicator (1) is provided with a closing means (4) to close off an access (15) to said cooling chamber (13), at least when said foam part (2) is situated in said cooling position.

10. An assembly according to claim 8 or 9, wherein the shaping cavity (12) is arranged to compress a treatment end (3) of the foam part (2), in particular to a pointed shape, in said cooling position of the foam part (2).

11. An assembly according to any one of claims 8-10, wherein said foam part (2) has an open cell structure.

12. An assembly according to any one of claims 8-11, wherein said foam part (2), in an uncompressed condition, is substantially cylinder-shaped.

13. An assembly according to any one of claims 8-12, wherein the cooling device (11) is provided with a fluid reservoir with releasing means (16, 17) for releasing cooling fluid to said cooling chamber (10).

14. An assembly according to any one of claims 8-13, wherein the cooling fluid comprises at least dimethyl ether and an alkane, in particular propane.

15. An assembly according to any one of claims 8-14, provided with at least two applicators (1; 201; 301) of different dimensions to provide, during use, foam parts (2; 202; 302) of different respective treatment shapes.

16. A cooling device evidently intended and suitable for an assembly according to any one of claims 8-15.
